# EUROPEAN PATENT SPECIFICATION

(11) **EP 4 403 121 B1**
(45) Date of publication and mention of the grant of the patent: **14.10.2026**
(21) Application number: 22885885.8
(22) Date of filing: 25.10.2022
(51) Int. Cl.: A61B 17/32, H02J 7/82, A61B 18/20, A61B 18/14, A61B 18/12

(54) **SURGICAL ELECTRIC INSTRUMENT AND ULTRASONIC SCALPEL**
CHIRURGISCHES ELEKTRISCHES INSTRUMENT UND ULTRASCHALLSKALPELL
INSTRUMENT ÉLECTRIQUE CHIRURGICAL ET SCALPEL À ULTRASONS

(30) Priority: 28.10.2021 CN 202111264221
(43) Date of publication of application: 24.07.2024
(73) Proprietor: Ensurge Medical (Suzhou) Co., Ltd., Suzhou, Jiangsu 215000 (CN)
(72) Inventor: ZHANG, Jun, Suzhou, Jiangsu 215000 (CN); WU, Zhixin, Suzhou, Jiangsu 215000 (CN)
(74) Representative: De Vries & Metman
(86) International application number: PCT/CN2022/127175
(87) International publication number: WO 2023/072008

(56) References cited:
- CN-A- 111 372 637
- CN-A- 112 535 517
- CN-A- 112 535 517
- CN-A- 113 855 175
- CN-A- 115 054 322
- CN-U- 207 804 334
- CN-U- 211 187 449
- JP-A- 2021 508 557
- US-A- 5 400 267
- US-A1- 2020 268 408

## Description

### Field

The present disclosure relates to the technical field of medical devices, and in particular, to an energy instrument for surgery and an ultrasonic scalpel.

### Background

Energy instruments for surgery, such as ultrasonic scalpels that use ultrasonic energy, are widely used in various endoscopic and conventional surgeries. Before surgery, medical staffs of the equipment department in hospital need to prepare surgical equipment and consumables for the surgery, and if energy instruments are involved, it is generally necessary to confirm their service life.

In order to record the remaining usable times of energy instruments, energy instruments with usage count statistical functions have been developed in the prior art, such as a Chinese utility model patent published as CN211187449U, which discloses an ultrasonic scalpel provided with an information recording device to record the usage times and remaining usable times of the transducer, and further provided with a display device to display various information received from an information recording device for reference by surgical operators. And it also discloses that when the usage count recorded by the information recording device is greater than or equal to a preset usage count threshold, it issues a warning message.

However, in the prior art, to check the device status, such as the number of usages, it is necessary to connect an external power supply to start the ultrasonic scalpel, and the wiring operation is relatively troublesome; there is also an energy instrument for ultrasonic scalpel in prior art, such as a Chinese patent published as CN207804334U, which discloses a cableless ultrasonic surgical system, namely providing an energy storage device inside the driving handle to power the ultrasonic driver, so that the ultrasonic scalpel can be used without external power supply, however, due to its principle, although it eliminates the complicated operation of connecting power wires, power loss is caused during the non-surgical stage by connecting the built-in battery to activate the ultrasonic scalpel to check the device status. Moreover, cableless ultrasonic surgery systems are relatively expensive, and the battery life and the stability of output power cannot be compared with wired ultrasonic surgery systems using external alternating current power, therefore, currently wired ultrasonic surgery systems are still mainstream instrument products.

US2020/0268408A1 discloses a surgical instrument including a battery, which is configured to discharge from an initial charge to a depleted charge within a desired time frame. This allows instrument to have a strictly enforced lifespan, beginning from the first time a connection is made with generator. This lifespan may be long enough to allow a lengthy surgical procedure to be completed, but short enough to greatly reduce the likelihood of overuse or abuse of instrument.

US5400267A discloses an equipment, such as surgical instrument, including control module and non-volatile memory. When equipment is energized, control module reads the aforementioned utilization limits and the accumulated utilization history stored in non-volatile memory, control module may use the accumulated utilization history and the utilization limits to disable equipment once the manufacturer recommended utilization limits have been exceeded.

### Summary

The energy instrument for surgery according to the invention is defined in claim 1. Embodiments are defined in the dependent claims.

The purpose of the present disclosure is to provide an energy instrument for surgery and an ultrasonic scalpel that can check device condition parameters with low power consumption without external power supply.

To achieve the above purpose, a technical solution employed by the present disclosure is:
An energy instrument for surgery with a dual-mode power supply circuit, comprises a built-in battery, a control unit, a first power-consumption circuit and a second power-consumption circuit, wherein the first power-consumption circuit is electrically connected to the control unit, and power consumption of the second power-consumption circuit in a working state is greater than power consumption of the first power-consumption circuit in a working state;
when the energy instrument for surgery is not connected to an external power supply, the control unit and the first power-consumption circuit can be triggered to respectively connect a loop with the built-in battery, and the first power-consumption circuit is configured to output information of a condition parameter of the energy instrument for surgery in response to an instruction of the control unit;
the second power-consumption circuit stays in stop working state when the energy instrument for surgery is not connected to an external power supply;
the energy instrument for surgery further comprises an operation key and a switch circuit, wherein the switch circuit comprises a first power switching tube and a second power switching tube with an initial state both in a switched-off condition, wherein the first power switching tube is arranged between the first power-consumption circuit and a negative electrode of the built-in battery, and the second power switching tube is arranged between the first power-consumption circuit and a positive electrode of the built-in battery;
a control terminal of the first power switching tube is connected to the positive electrode of the built-in battery through the operation key, and a control terminal of the second power switching tube is connected to the first power switching tube; in response to a triggering of the operation key, both the first power switching tube and the second power switching tube are converted from the switched-off condition to a switched-on condition;
the second power-consumption circuit is neither connected to the positive nor to negative electrode of the built-in battery.

Specifically, the energy instrument for surgery may be connected to an external power supply through a power connector, and the control terminal of the first power switching tube is connected to the built-in battery through the operation key, and is configured to connect both the control unit and the first power-consumption circuit to the negative electrode of the built-in battery if the first power switching tube is switched on;
the control terminal of the second power switching tube is connected to one end of the first power switching tube, and is configured to connect both the control unit and the first power-consumption circuit to the positive electrode of the built-in battery if the second power switching tube is switched on;
the second power-consumption circuit is neither connected to the positive nor to negative electrode of the built-in battery;
if the energy instrument for surgery is disconnected from the external power supply and the operation key is pressed, the built-in battery triggers a conducting of the first power switching tube, and then the conducting of the first power switching tube triggers a conducting of the second power switching tube, so that the control unit and the first power-consumption circuit enter working state.

Further, the first power-consumption circuit is any one of or a combination of:
the first power-consumption circuit is a display unit configured to display the condition parameter of the energy instrument for surgery under the control of the control unit; and/or
the first power-consumption circuit is a loudspeaker unit configured to play voice messages under the control of the control unit, which reflect the condition parameter of the energy instrument for surgery; and/or
the first power-consumption circuit is an indicator light device configured to light up under the control of a control unit to reflect the condition parameter of the energy instrument for surgery.

Further, the switch circuit further comprises a third power switching tube and a fourth power switching tube with an initial state both in a switched-off condition, wherein the third power switching tube is arranged between the second power-consumption circuit and the negative electrode of the external power supply, and the fourth power switching tube is arranged between the second power-consumption circuit and a positive electrode of the external power supply;
a control terminal of the third power switching tube is connected to the external power supply through a power connector, and a control terminal of the fourth power switching tube is connected to the third power switching tube; in response to the connection of the energy instrument for surgery to the external power supply, both the third power switching tube and the fourth power switching tube are converted from the switched-off condition to a switched-on condition. Specifically, if the third power switching tube is switched on, the second power-consumption circuit is connected to the negative electrode of the external power supply;
the control terminal of the fourth power switching tube is connected to one end of the third power switching tube, and is configured to connect both the second power-consumption circuit and the control unit to the positive electrode of the external power supply if the third power switching tube is switched on;
if the energy instrument for surgery is connected to the external power supply, a conducting of the third power switching tube is triggered, and then the conducting of the third power switching tube triggers a conducting of the fourth power switching tube, so that the control unit and the second power-consumption circuit are in the working state.

Further, the second power-consumption circuit is a drive circuit or a digital processing circuit.

Further, the operation key is a button dedicated to controlling a startup of the first power-consumption circuit; or,
the operation key is a function key of the energy instrument for surgery, which has a first working mode and a second working mode, if the energy instrument for surgery is disconnected from an external power supply, the control unit controls the function key to work in the first working mode, including: if the function key is pressed, triggering the conducting of the first power switching tube; if the energy instrument for surgery is connected to an external power supply, the control unit controls the function key to work in the second working mode, including: if the function key is pressed, activating another function different from triggering the conducting of the first power switching tube.

Further, another function different from triggering the conducting of the first power switching tube comprises any one of the following functions:
the function key is an excitation button, and if the excitation button is pressed, it will activate an energy generator of the energy instrument for surgery to generate energy; or,
the function key is a power shift button, and if the power shift button is pressed, it will adjust an output power of an energy generator of the energy instrument for surgery; or,
the function key is a knife head control button, and if the knife head control button is pressed, it will control a cutting tool end of the energy instrument for surgery to perform a clamping action.

Further, the energy instrument for surgery with a dual-mode power supply circuit further comprises a timer electrically connected to the control unit; when the energy instrument for surgery is not connected to an external power supply, in response to a triggering of the first power-consumption circuit, the timer starts timing, and when a preset delay threshold is reached, the first power-consumption circuit is disconnected from the loop with the built-in battery;

Specifically, if the energy instrument for surgery is disconnected from the external power supply and the operation key is pressed, the timer starts timing and when the preset delay threshold is reached, the control unit sends a low level to the control terminal of the first power switching tube to convert the first power switching tube from the switched-on condition to the switched-off condition.

Further, the operation key is configured to:
if the operation key is being pressed, the first power switching tube remains in the switched-on condition;
if the operation key is released, the first power switching tube is converted from the switched-on condition to the switched-off condition.

Further, the control terminals of the second power switching tube and the fourth power switching tube are both connected to the positive electrode of the built-in battery through a resistor, and in the initial state, the second power switching tube and the fourth power switching tube are both in the switched-off condition due to that their gate voltages are both higher than a conducting voltage threshold;
in response to the conducting of the first power switching tube, the second power switching tube is switched on due to its gate voltage is pulled down; in response to the conducting of the third power switching tube, the fourth power switching tube is switched on due to its gate voltage is pulled down.

Further, the second power switching tube and the fourth power switching tube are both P-type Mos tubes, and the first power switching tube and the third power switching tube are both N-type Mos tubes.

Further, the fourth power switching tube is connected to the control terminal of the first power switching tube through a resistor;
if the energy instrument for surgery is connected to an external power supply, the conducting of the fourth power switching tube triggers the conducting of the first power switching tube, so that the control unit is connected to the negative electrode of the external power supply.

Further, the built-in battery is a rechargeable battery, and if the energy instrument for surgery is connected to an external power supply, the rechargeable battery is connected to the external power supply through the fourth power switching tube;
or, the built-in battery is a non-rechargeable battery.

Further, the control unit is connected to a gate of the third power switching tube, and in response to a connection of the built-in battery or an external power supply, the control unit detects a gate voltage of the third power switching tube and determines whether the power connector is connected to the external power supply by the gate voltage of the third power switching tube.

Further, the energy instrument for surgery is ultrasonic scalpel, electric scalpel, or laser scalpel.

The beneficial effects brought by the technical solutions provided by the present disclosure are as follows:
a. In the case where the energy instrument for surgery is not connected to an external power supply, the low-power consumption circuit can be triggered by pressing an operation key, in such configuration, staff of the equipment department in hospital can quickly and conveniently query the status of the equipment, such as the remaining usable times or capacity value;
b. It ensures that the high-power consumption circuits are not started by the built-in battery, which not only reduces the requirement for battery capacity but also reduces the energy consumption of battery power.

### Brief Description of the Drawings

For more clearly explaining the technical solutions in the embodiments of the present application or in the prior art, the accompanying drawings required to be used to in the description of the embodiments or the prior art will be simply introduced below. Apparently, the drawings in the following description show merely some embodiments described in the present application, and those of ordinary skill in the art may derive other drawings from these accompanying drawings without creative efforts.

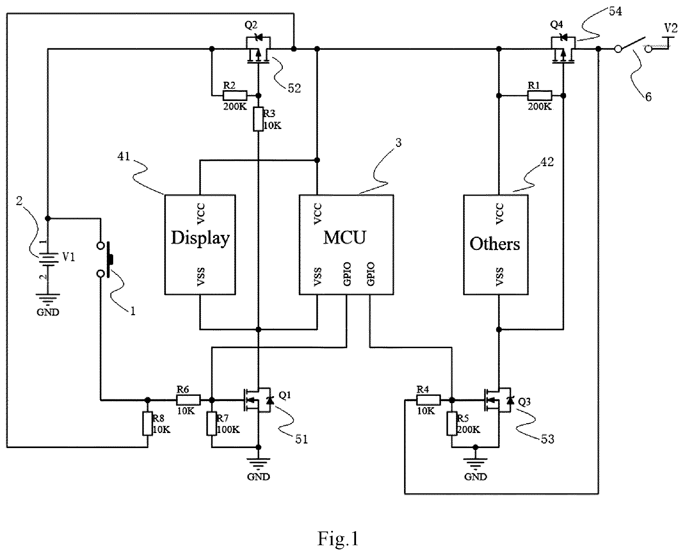

Figure 1 is a schematic structure diagram of an energy instrument for surgery with a dual-mode power supply circuit provided in an exemplary embodiment of the present disclose;
wherein, reference signs comprise: 1 - operation key, 2 - built-in battery, 3 - control unit, 41 - first power-consumption circuit, 42 - second power-consumption circuit, 51 - first power switching tube, 52 - second power switching tube, 53 - third power switching tube, 54 - fourth power switching tube, 6 - power connector.

### Detailed Description

In order to make those skilled in the art better understand the solutions of the present disclosure, the technical solutions in the embodiments of the present disclosure will be described clearly and completely below with reference to the accompanying drawings in the embodiments of the present disclosure. Apparently, the described embodiments are merely some of rather than all of the embodiments of the present disclosure. All other embodiments obtained by those of ordinary skill in the art without creative efforts based on the embodiments of the present disclosure shall fall within the protective scope of the present disclosure.

It should be noted that terms such as "first" and "second" in the description, the claims and the accompanying drawings of the present disclosure are used to/intended to distinguish similar objects, and do not have to be used to/are not intended to describe a specific order or sequence. It should be understood that data used in this manner may be interchangeable where appropriate, so that the embodiments of the present disclosure described herein can be implemented in an order other than/in addition to those illustrated or described herein. In addition, terms "comprise" and "have" and any other variants thereof are intended to cover non-exclusive inclusion, for example, a process, method, device, product, or equipment that includes a series of steps or units is not necessarily limited to those clearly listed steps or units, but may include other steps or units not listed clearly or inherent to the process, method, product, or equipment.

Before surgery, medical staff of the equipment department in hospital need to prepare surgical equipment and consumables for the surgery, and if energy instrument is involved, it is generally necessary to confirm its service life to prevent the remaining usable times of energy instrument from being found to be zero after surgery has begun. In this regard, the traditional solution is to manually record the remaining usable times of each energy instrument during each postoperative equipment sorting, and a more advanced approach may be that the energy instruments themselves have a statistical function for the remaining usage times, which can be queried in real-time by the equipment department. However, the responsibilities of each process within the hospital are clear, and the doctors performing the surgeries have no obligation to query and record the remaining usable times before stopping the operations, which requires the equipment department to operate a query for the remaining usable times of energy instruments after being powered on during preoperative examinations. It is not very convenient to either manually record the remaining usable times or to query it after powering on, and the concept of the present disclosure is to provide a solution for convenient querying the remaining usable times of energy instruments for surgery without connecting to an external power source.

In one embodiment of the present disclosure, an energy instrument for surgery with a dual-mode power supply circuit is provided, which is configured to be connected to an external power supply through a power connector 6, as shown in Figure 1, the energy instrument for surgery comprises an operation key 1, a built-in battery 2, a control unit 3, a first power-consumption circuit 41, a second power-consumption circuit 42, wherein the second power-consumption circuit 42 is a drive circuit or a digital processing circuit, wherein, the drive circuit can be configured to drive an energy generator, namely a power amplifier module of an energy generator of a transducer, and can also be configured to drive a switching tube or a motor; the digital processing circuit can be a CPU or DSP or other digital circuit. The power consumption of the first power-consumption circuit 41 is lower than the power consumption of the second power-consumption circuit 42, and the first power-consumption circuit 41 is configured to prompt the condition parameter of the energy instrument for surgery under the control of the control unit 3, and taking the remaining usable times of the instrument as the condition parameter as an example, several forms of presentation of the first power-consumption circuit 41 are described below:
The first form is that the first power-consumption circuit is a display unit, such as a liquid crystal display screen, which is configured to display the condition parameter of the energy instrument for surgery under the control of the control unit;
The second form is that the first power-consumption circuit is a loudspeaker unit, which is configured to play voice messages under the control of the control unit, such as "the current remaining usable times are 7";
The third form is that the first power-consumption circuit is an indicator light device, which can comprises multiple colors thereof, for example, a green light indicates that the current remaining usable times are greater than or equal to 20 times, a yellow light indicates that the current remaining usable times are greater than or equal to 3 times and less than 20 times, and a red light indicates that the current remaining usable times are less than 3 times; the present disclosure does not limit the colors that reflect the condition parameter of the energy instrument for surgery, for example, different brightness of the indicator light can also be used to reflect the current remaining usable times, with brighter indicating more times and weaker indicating fewer times; for example, different indicator lights can be marked with '>20', '[5-20]', '<5', and the indicator lights in the corresponding area can be lit based on the actual remaining usable times, and so on.

The remaining usable times can be recorded by setting up an information recording device to record the actual usage times of the transducer, as mentioned in the prior art CN211187449U in the section of 'Background', and the remaining usable times can be calculated; when the control unit and the first power-consumption circuit are triggered without connecting to an external power supply, the control unit queries the information recording device for the remaining usable times and sends the query results to the first power-consumption circuit to achieve a prompt function.

In order to drive the first power-consumption circuit 41 without connecting to an external power source, the energy instrument for surgery further comprises a switch circuit, in the present embodiment, the switch circuit may be designed as follows:
Referring to Figure 1, the switch circuit comprises a first power switching tube 51 (i.e., the switching tube Q1 in Figure 1), a second power switching tube 52 (i.e., the switching tube Q2 in Figure 1), a third power switching tube 53 (hereinafter referred to as switching tube Q3), and a fourth power switching tube 54 (hereinafter referred to as switching tube Q4) with an initial state all in a switched-off condition, wherein a control terminal of the switching tube Q1 is connected to the built-in battery through the operation key, and is configured to connect both the control unit and the first power-consumption circuit to the negative electrode of the built-in battery if the switching tube Q1 is switched on;

A control terminal of the switching tube Q2 is connected to one end of the switching tube Q1, and is configured to connect both the control unit 3 and the first power-consumption circuit 41 to the positive electrode of the built-in battery 2 if the switching tube Q2 is switched on;

A control terminal of the switching tube Q3 is connected to the external power supply through the power connector 6, and is configured to connect the second power-consumption circuit 42 to the negative electrode of the external power supply if the switching tube Q3 is switched on;

A control terminal of the switching tube Q4 is connected to one end of the switching tube Q3, and is configured to connect both the second power-consumption circuit 42 and the control unit 3 to the positive electrode of the external power supply if the switching tube Q3 is switched on;

It can be seen from Figure 1 that the second power-consumption circuit 42 is neither connected to the positive electrode nor to the negative electrode of the built-in battery 2.

Taking Figure 1 as an example, the switching tube Q1 and the switching tube Q3 are N-type Mos tubes, and switching characteristic thereof is that when the gate voltage is higher than a certain voltage value, the switching tube is switched on, otherwise it is in the cut-off condition; the switching tube Q2 and the switching tube Q4 are P-type Mos tubes, and switching characteristic thereof is that when the gate voltage is lower than a certain voltage value, the switching tube is switched on, otherwise it is in a cut-off condition;

The control terminal of the switching tube Q2 is connected to the positive electrode of the built-in battery 2 through a resistor R2 in Figure 1, and the control terminal of the switching tube Q4 is connected to the positive electrode of the built-in battery 2 through a resistor R1 in Figure 1, so that their gate voltages are higher than a conducting voltage threshold, at this time, the initial state of the switching tubes Q2 and Q4 is the cut-off condition (switched-off condition);

In the initial state, the gate of switching tube Q1 is grounded through a resistor R7 and is at a low level, and there are two approaches to pulled up its gate voltage, which is described as below:
The first approach is that when the energy instrument for surgery is disconnected from the external power supply, pressing the operation key 1 can cause the positive electrode of the built-in battery 2 to provide a high voltage to the gate of the switching tube Q1 to switch it on, where the operation key 1 may be selected to be a key which makes a circuit conduct by being pressed by an external force, if the operation key is pressed, the switching tube Q1 remains being switched on; if the operation key is released, the switching tube Q1 is converted from the switched-on condition to a switched-off condition. In this way, medical staff in the equipment department can hold the operation key 1 pressed when querying the remaining usable times to have enough time to record the corresponding query results; or, at a delay of a period of time, such as 10 seconds, after the operation key has been pressed, the control unit sends a low level to the control terminal of the switching tube Q1, so that the switching tube Q1 is converted from the switched-on condition to the switched-off condition, in this case, an additional timer electrically connected to the control unit needs to be provided, but it is not required to keep pressing the operation key 1 manually.
The second approach is that when the energy instrument for surgery is connected to an external power supply, firstly, the gate voltage of the switching tube Q3 is pulled up due to the influence of the connection of the external power supply, after the switching tube Q3 is switched on, the gate of the switching tube Q4 is grounded through the switched-on switching tube Q3, that is, the gate voltage of the switching tube Q4 is pulled down, and then the switching tube Q4 is switched on successively; further, the external power supply pulls up the gate voltage of the switching tube Q1 through the switched-on switching tube Q4 and resistors (see R8 and R6 in Figure 1), and then the switching tube Q1 is switched on.

If the switching tube Q1 is switched on, the switching tube Q2 is switched on due to its gate voltage is pulled down.

The above provides a detailed explanation of the triggering and conducting relationships between various power switching tubes in the switch circuit.

The above switching tubes Q4 - Q1 can achieve that when powered by the built-in battery, only the low-power circuit can be triggered to work by the operation key 1, while the power amplifier circuit of the energy generator does not power on (do not work), which can not only check the device condition parameter for a short time, but also would not bring excessive loss to the battery due to not accessing to the high-power consumption circuit, extending the service life of the battery without the need for frequent battery replacement during the life time of the instrument (in the case of non-rechargeable batteries), and a small-capacity battery can meet the demand; when powered by an external power supply, all circuits (including the high-power consumption circuit and the low-power consumption circuit) are powered on (entering the working state).

That is to say, in the case that the energy instrument for surgery is disconnected from the external power supply, when the operation key 1 is pressed, the built-in battery triggers the conducting of the switching tube Q1, and then the conducting of the switching tube Q1 triggers the conducting of the switching tube Q2, so that the control unit and the first power-consumption circuit 41 are in the working state, at this time the control unit can send the pre-set device condition parameter to the first power-consumption circuit 41, and then the first power-consumption circuit 41 will make a prompt action. Please refer to the relevant solutions of the display screen, loudspeaker or indicator light mentioned above, which will not be repeated here.

When the energy instrument for surgery is connected to the external power supply, the switching tube Q3 is switched on due to that the gate voltage of the switching tube Q3 is pulled up by the external power supply, and then the conducting of the switching tube Q3 triggers the conducting of the switching tube Q4, so that the VCC ends of the control unit 3, the second power-consumption circuit 42 and the first power-consumption circuit 41 are all connected to the positive electrode of the external power supply. As mentioned above, the conducting of the switching tube Q4 will trigger the conducting of the switching tube Q1, then the VSS ends of the first power-consumption circuit 41 and the control unit 3 are connected to the negative electrode of the external power supply (grounded), at this time, the control unit 3, the second power-consumption circuit 42 and the first power-consumption circuit 41 all enter the working state.

Referring to Figure 1, the control unit 3 is also connected to the gate of the switching tube Q3, and in response to the connection of the built-in battery or an external power supply, the control unit detects the gate voltage of the switching tube Q3 and determines whether the power connector 6 is connected to the external power supply by the gate voltage of the switching tube Q3, specifically, if the gate pin level of switching tube Q3 is high, it indicates that the instrument is currently powered by an external power supply, and if it is low, it indicates that the instrument is currently powered by the built-in battery. The present disclosure does not impose specific limitations on the specific built-in position of the built-in battery.

The control unit 3 is also connected to the gate of the switching tube Q1, and determines whether the operation key 1 is pressed by the voltage change of the gate of the switching tube Q1, specifically, if it is determined from the previous description whether it is powered by an external power supply or the built-in battery, and in the case that it is powered by the built-in battery, if the gate pin level of the switching tube Q1 changes from low to high, it indicates that the operation key 1 is currently pressed; in the case that it is powered by the external power supply, if the gate level amplitude of the switching tube Q1 is further increased, it indicates that the operation key 1 is currently pressed.

There are two different implementations for the operating key:
The first implementation is that the operation key is a button dedicated to controlling the start of the first power-consumption circuit, that is, adding a new button on the basis of the operation key of the existing energy instrument for surgery;
The second implementation is to not add a new button on the basis of the operation keys of the existing energy instrument for surgery, and one or more function keys can be selected as the start button to trigger a startup of the first power-consumption circuit 41, in this way, the operation key (the selected function key(s)) has a first working mode and a second working mode, if the energy instrument for surgery is disconnected from an external power supply, the control unit controls the function key(s) to work in the first working mode, including: if the function key(s) is pressed, triggering the conducting of the switching tube Q1; if the energy instrument for surgery is connected to an external power supply, the control unit controls the function key(s) to work in the second working mode, including: if the function key(s) is pressed, activating another function different from triggering the conducting of the switching tube Q1. Specifically, another function different from triggering the conducting of the switching tube Q1 comprises any one of the following functions:
   the function key is an excitation button, and if the excitation button is pressed, it will activate an energy generator of the energy instrument for surgery to generate energy; or,
   the function key is a power shift button, and if the power shift button is pressed, it will adjust the output power of the energy generator of the energy instrument for surgery; or,
   the function key is a knife head control button, and if the knife head control button is pressed, it will control the cutting tool end of the energy instrument for surgery to perform a clamping action. The present disclosure does not limit the entire selection range as the excitation button, the power shift button, and knife head control button mentioned above, and all the existing function keys on energy instruments can be selected and have the first mode and second working mode.

In one embodiment of the present disclosure, the built-in battery is a rechargeable battery, and if the energy instrument for surgery is connected to an external power supply, the rechargeable battery is connected to the external power supply through the switching tube Q4; specifically, as mentioned above, in response to the connection of the energy instrument for surgery to the external power supply, the switching tube Q3 is switched on, and then the switching tubes Q4, Q1, and Q2 are switched on successively, at this time, the external power supply charges the rechargeable battery through the switching tubes Q4 and Q2 in the switched-on condition.

The present disclosure does not impose specific limitations on the energy instrument for surgery, in one embodiment of the present disclosure, the energy instrument for surgery is a ultrasonic scalpel, electric scalpel, or laser scalpel.

It should be noted that herein, relational terms such as first and second are only used to distinguish one entity or operation from another entity or operation, and do not necessarily require or imply any actual relationship or order between these entities or operations. Moreover, terms "comprising", "including", or any other variation thereof are intended to encompass non-exclusive inclusion, such that a process, method, item, or device that includes a series of elements not only includes those elements, but also includes other elements that are not explicitly listed, or also include elements inherent in such a process, method, item, or device. Without further limitations, elements limited by the statement "comprising a..." do not exclude the existence of other identical elements in the process, method, item, or device that includes the elements.

The above are only specific implementations of the present application. It should be noted that, for those ordinary skilled/of ordinary skill in the art, any improvements or modifications can be made without depart from the technical principle and conception of the present disclosure.

## Claims

1. An energy instrument for surgery, it comprises a built-in battery (2), a control unit (3), a first power-consumption circuit (41) and a second power-consumption circuit (42), wherein the first power-consumption (41) circuit is electrically connected to the control unit (3), and power consumption of the second power-consumption circuit (42) in a working state is greater than power consumption of the first power-consumption circuit (41) in a working state;
wherein, when the energy instrument for surgery is not connected to an external power supply, the control unit (3) and the first power-consumption circuit (41) can be triggered to respectively connect a loop with the built-in battery (2), and the first power-consumption circuit (41) is configured to output information of a condition parameter of the energy instrument for surgery in response to an instruction of the control unit (3);
the second power-consumption circuit (42) is configured to stay in stop working state when the energy instrument for surgery is not connected to an external power supply;
the energy instrument for surgery further comprises an operation key (1) and a switch circuit, wherein the switch circuit comprises a first power switching tube (51) and a second power switching tube (52) with an initial state both in a switched-off condition, wherein the first power switching tube (51) is arranged between the first power-consumption circuit (41) and a negative electrode of the built-in battery (2), and the second power switching tube (52) is arranged between the first power-consumption circuit (41) and a positive electrode of the built-in battery (2);
a control terminal of the first power switching tube (51) is connected to the positive electrode of the built-in battery (2) through the operation key (1), and a control terminal of the second power switching tube (52) is connected to the first power switching tube (51); in response to a triggering of the operation key (1), both the first power switching tube (51) and the second power switching tube (52) are configured to be converted from the switched-off condition to a switched-on condition;
the second power-consumption circuit (42) is neither connected to the positive electrode nor to negative electrode of the built-in battery (2).

2. The energy instrument for surgery according to claim 1, **characterized in that**, the first power-consumption circuit (41) is any one of or a combination of:
the first power-consumption circuit (41) is a display unit configured to display the condition parameter of the energy instrument for surgery under the control of the control unit (3); and/or
the first power-consumption circuit (41) is a loudspeaker unit configured to play voice messages under the control of the control unit (3), which reflect the condition parameter of the energy instrument for surgery; and/or
the first power-consumption circuit (41) is an indicator light device configured to light up under the control of a control unit (3) to reflect the condition parameter of the energy instrument for surgery.

3. The energy instrument for surgery according to claim 1, **characterized in that**, the switch circuit further comprises a third power switching tube (53) and a fourth power switching tube (54) with an initial state both in a switched-off condition, wherein the third power switching tube (53) is arranged between the second power-consumption circuit (42) and a negative electrode of the external power supply, and the fourth power switching tube (54) is arranged between the second power-consumption circuit (42) and a positive electrode of the external power supply;
a control terminal of the third power switching tube (53) is connected to the external power supply through a power connector (6), and a control terminal of the fourth power switching tube (54) is connected to the third power switching tube (53); in response to the connection of the energy instrument for surgery to the external power supply, both the third power switching tube (53) and the fourth power switching tube (54) are converted from the switched-off condition to a switched-on condition.

4. The energy instrument for surgery according to claim 3, **characterized in that**, the second power-consumption circuit (42) is a drive circuit or a digital processing circuit.

5. The energy instrument for surgery according to claim 1, **characterized in that**, the operation key (1) is a button dedicated to controlling a startup of the first power-consumption circuit (41); or,
the operation key (1) is a function key of the energy instrument for surgery, if the energy instrument for surgery is disconnected from the external power supply, and the function key is pressed, the first power switching tube (51) is triggered for conducting; if the energy instrument for surgery is connected to the external power supply, and the function key is pressed, another function different from triggering the first power switching tube (51) for conducting is activated.

6. The energy instrument for surgery according to claim 5, **characterized in that**, another function different from triggering the first power switching tube (51) for conducting comprises any one of the following functions:
the function key is an excitation button, and if the excitation button is pressed, it will activate an energy generator of the energy instrument for surgery to generate energy; or,
the function key is a power shift button, and if the power shift button is pressed, it will adjust an output power of an energy generator of the energy instrument for surgery; or,
the function key is a knife head control button, and if the knife head control button is pressed, it will control a cutting tool end of the energy instrument for surgery to perform a clamping action.

7. The energy instrument for surgery according to claim 1, **characterized in that**, it further comprises a timer electrically connected to the control unit (3);
when the energy instrument for surgery is not connected to an external power supply, in response to a triggering of the first power-consumption circuit (41), the timer starts timing, and when a preset delay threshold is reached, the first power-consumption circuit (41) is disconnected from the loop with the built-in battery (2).

8. The energy instrument for surgery according to claim 1, **characterized in that**, the operation key (1) is configured to:
if the operation key is being pressed, the first power switching tube (51) remains in the switched-on condition;
if the operation key (1) is released, the first power switching tube (51) is converted from the switched-on condition to the switched-off condition.

9. The energy instrument for surgery according to claim 3, **characterized in that**, the control terminals of the second power switching tube (52) and the fourth power switching tube (54) are both connected to the positive electrode of the built-in battery (2) through a resistor, and in the initial state, the second power switching tube (52) and the fourth power switching tube (54) are both in the switched-off condition due to that gate voltages thereof are both higher than a conducting voltage threshold;
in response to a conducting of the first power switching tube (51), the second power switching tube (52) is switched on due to gate voltage thereof is pulled down; in response to a conducting of the third power switching tube (53), the fourth power switching tube (54) is switched on due to gate voltage thereof is pulled down.

10. The energy instrument for surgery according to claim 9, **characterized in that**, the second power switching tube (52) and the fourth power switching tube (54) are both P-type Mos tubes, and the first power switching tube (51) and the third power switching tube (53) are both N-type Mos tubes.

11. The energy instrument for surgery according to claim 3, **characterized in that**, the fourth power switching tube (54) is connected to the control terminal of the first power switching tube (51) through a resistor;
if the energy instrument for surgery is connected to an external power supply, a conducting of the fourth power switching tube (54) triggers a conducting of the first power switching tube (51), so that the control unit (3) is connected to the negative electrode of the external power supply.

12. The energy instrument for surgery according to claim 3, **characterized in that**, the built-in battery (2) is a rechargeable battery, and if the energy instrument for surgery is connected to an external power supply, the rechargeable battery is connected to the external power supply through the fourth power switching tube (54);
Or, the built-in battery (2) is a non-rechargeable battery.

13. The energy instrument for surgery according to claim 3, **characterized in that**, the control unit (3) is connected to a gate of the third power switching tube (53), and in response to a connection of the built-in battery (2) or an external power supply, the control unit (3) detects a gate voltage of the third power switching tube (53) and determines whether the power connector (6) is connected to the external power supply by the gate voltage of the third power switching tube (53).

14. The energy instrument for surgery according to any one of claims 1 - 13, **characterized in that**, the energy instrument for surgery is ultrasonic scalpel, electric scalpel, or laser scalpel.

## Patentansprüche

1. Ein Energieinstrument für die Chirurgie, das eine eingebaute Batterie (2), eine Steuereinheit (3), eine erste Stromverbrauchsschaltung (41) und eine zweite Stromverbrauchsschaltung (42) aufweist, wobei die erste Stromverbrauchsschaltung (41) elektrisch mit der Steuereinheit (3) verbunden ist, und der Stromverbrauch der zweiten Stromverbrauchsschaltung (42) in einem Betriebszustand größer ist als der Stromverbrauch der ersten Stromverbrauchsschaltung (41) in einem Betriebszustand;
wobei, wenn das Energieinstrument für die Chirurgie nicht an eine externe Stromversorgung angeschlossen ist, die Steuereinheit (3) und die erste Stromverbrauchsschaltung (41) dazu veranlasst werden können, sich jeweils mit der eingebauten Batterie (2) zu einem Stromkreis zu verbinden, und die erste Stromverbrauchsschaltung (41) so konfiguriert ist, dass sie als Reaktion auf einen Befehl der Steuereinheit (3) Informationen über einen Zustandsparameter des Energieinstruments für die Chirurgie ausgibt;
die zweite Stromverbrauchsschaltung (42) so konfiguriert ist, dass sie in einem Nichtbetriebszustand verbleibt, wenn das Energieinstrument für die Chirurgie nicht an eine externe Stromversorgung angeschlossen ist;
das Energieinstrument für die Chirurgie ferner eine Bedientaste (1) und einen Schaltschaltkreis aufweist, wobei der Schaltschaltkreis eine erste Stromschaltröhre (51) und eine zweite Stromschaltröhre (52) aufweist, die sich in einem Ausgangszustand beide in einem ausgeschalteten Zustand befinden, wobei die erste Stromschaltröhre (51) zwischen der ersten Stromverbrauchsschaltung (41) und einer negativen Elektrode der eingebauten Batterie (2) angeordnet ist und die zweite Stromschaltröhre (52) zwischen der ersten Stromverbrauchsschaltung (41) und einer positiven Elektrode der eingebauten Batterie (2) angeordnet ist;
ein Steueranschluss der ersten Stromschaltröhre (51) über die Bedientaste (1) mit der positiven Elektrode der eingebauten Batterie (2) verbunden ist und ein Steueranschluss der zweiten Stromschaltröhre (52) mit der ersten Stromschaltröhre (51) verbunden ist; als Reaktion auf ein Auslösen der Bedientaste (1) sowohl die erste Stromschaltröhre (51) als auch die zweite Stromschaltröhre (52) so konfiguriert sind, dass sie von dem ausgeschalteten Zustand in einen eingeschalteten Zustand versetzt werden;
die zweite Stromverbrauchsschaltung (42) weder mit der positiven Elektrode noch mit der negativen Elektrode der eingebauten Batterie (2) verbunden ist.

2. Das Energieinstrument für die Chirurgie nach Anspruch 1, **dadurch gekennzeichnet, dass** die erste Stromverbrauchsschaltung (41) irgendeines ist von oder eine Kombination von:
die erste Stromverbrauchsschaltung (41) ist eine Anzeigeeinheit, die so konfiguriert ist, dass sie gesteuert durch die Steuereinheit (3) den Zustandsparameter des Energieinstruments für die Chirurgie anzeigt; und/oder
die erste Stromverbrauchsschaltung (41) ist eine Lautsprechereinheit, die so konfiguriert ist, dass sie gesteuert durch die Steuereinheit (3) Sprachmeldungen wiedergibt, die den Zustandsparameter des Energieinstruments für die Chirurgie widerspiegeln; und/oder
die erste Stromverbrauchsschaltung (41) ist eine Indikatorlichtvorrichtung, die so konfiguriert ist, dass sie gesteuert durch die Steuereinheit (3) aufleuchtet, um den Zustandsparameter des Energieinstruments für die Chirurgie widerzuspiegeln.

3. Das Energieinstrument für die Chirurgie nach Anspruch 1, **dadurch gekennzeichnet, dass** der Schaltschaltkreis ferner eine dritte Stromschaltröhre (53) und eine vierte Stromschaltröhre (54) aufweist, die sich in einem Ausgangszustand beide in einem ausgeschalteten Zustand befinden, wobei die dritte Stromschaltröhre (53) zwischen der zweiten Stromverbrauchsschaltung (42) und einer negativen Elektrode der externen Stromversorgung angeordnet ist und die vierte Stromschaltröhre (54) zwischen der zweiten Stromverbrauchsschaltung (42) und einer positiven Elektrode der externen Stromversorgung angeordnet ist;
ein Steueranschluss der dritten Stromschaltröhre (53) über einen Netzanschluss (6) mit der externen Stromversorgung verbunden ist und ein Steueranschluss der vierten Stromschaltröhre (54) mit der dritten Stromschaltröhre (53) verbunden ist;
als Reaktion auf die Verbindung des Energieinstruments für die Chirurgie mit der externen Stromversorgung sowohl die dritte Stromschaltröhre (53) als auch die vierte Stromschaltröhre (54) vom ausgeschalteten Zustand in einen eingeschalteten Zustand versetzt werden.

4. Das Energieinstrument für die Chirurgie nach Anspruch 3, **dadurch gekennzeichnet, dass** die zweite Stromverbrauchsschaltung (42) eine Ansteuerschaltung oder eine digitale Verarbeitungsschaltung ist.

5. Das Energieinstrument für die Chirurgie nach Anspruch 1, **dadurch gekennzeichnet, dass** die Bedientaste (1) eine Taste ist, die speziell zur Steuerung des Starts der ersten Stromverbrauchsschaltung (41) vorgesehen ist; oder
die Bedientaste (1) eine Funktionstaste des Energieinstruments für die Chirurgie ist, wenn das Energieinstrument für die Chirurgie von der externen Stromversorgung getrennt ist und die Funktionstaste gedrückt wird, die erste Stromschaltröhre (51) zum Leiten veranlasst wird; wenn das Energieinstrument für die Chirurgie an die externe Stromversorgung angeschlossen ist und die Funktionstaste gedrückt wird, eine andere Funktion als das Veranlassen der ersten Stromschaltröhre (51) zum Leiten aktiviert wird.

6. Das Energieinstrument für die Chirurgie nach Anspruch 5, **dadurch gekennzeichnet, dass** die andere Funktion, die sich vom Veranlassen der ersten Stromschaltröhre (51) zum Leiten unterscheidet, eine der folgenden Funktionen umfasst:
die Funktionstaste ist eine Erregungstaste, und wenn die Erregungstaste gedrückt wird, wird ein Energiegenerator des Energieinstruments für die Chirurgie aktiviert, um Energie zu erzeugen; oder
die Funktionstaste ist eine Leistungsumschalttaste, und wenn die Leistungsumschalttaste gedrückt wird, wird eine Ausgangsleistung eines Energiegenerators des Energieinstruments für die Chirurgie angepasst; oder
die Funktionstaste ist eine Schneidkopfsteuertaste, und wenn die Schneidkopfsteuertaste gedrückt wird, wird ein Schneidwerkzeugende des Energieinstruments für die Chirurgie so gesteuert, dass es eine Klemmwirkung ausübt.

7. Das Energieinstrument für die Chirurgie nach Anspruch 1, **dadurch gekennzeichnet, dass** es ferner einen Zeitnehmer aufweist, der elektrisch mit der Steuereinheit (3) verbunden ist;
wenn das Energieinstrument für die Chirurgie nicht an eine externe Stromversorgung angeschlossen ist, der Zeitnehmer als Reaktion auf ein Auslösen der ersten Stromverbrauchsschaltung (41) mit einer Zeitmessung beginnt, und wenn ein voreingestellter Verzögerungsschwellenwert erreicht ist, die erste Stromverbrauchsschaltung (41) vom Stromkreis mit der eingebauten Batterie (2) getrennt wird.

8. Das Energieinstrument für die Chirurgie gemäß Anspruch 1, **dadurch gekennzeichnet, dass** die Bedientaste (1) so konfiguriert ist, dass:
wenn die Bedientaste gedrückt wird, die erste Stromschaltröhre (51) im eingeschalteten Zustand verbleibt;
wenn die Bedientaste (1) losgelassen wird, die erste Stromschaltröhre (51) vom eingeschalteten Zustand in den ausgeschalteten Zustand versetzt wird.

9. Das Energieinstrument für die Chirurgie nach Anspruch 3, **dadurch gekennzeichnet, dass** die Steueranschlüsse der zweiten Stromschaltröhre (52) und der vierten Stromschaltröhre (54) beide über einen Widerstand mit der positiven Elektrode der eingebauten Batterie (2) verbunden sind und sich im Ausgangszustand die zweite Stromschaltröhre (52) und die vierte Stromschaltröhre (54) beide im ausgeschalteten Zustand befinden, da ihre Gate-Spannungen beide höher sind als ein Leitungsspannungsschwellenwert;
als Reaktion auf ein Leiten der ersten Stromschaltröhre (51) die zweite Stromschaltröhre (52) eingeschaltet wird, da deren Gate-Spannung nach unten gezogen wird; als Reaktion auf ein Leiten der dritten Stromschaltröhre (53) die vierte Stromschaltröhre (54) eingeschaltet wird, da deren Gate-Spannung nach unten gezogen wird.

10. Das Energieinstrument für die Chirurgie nach Anspruch 9, **dadurch gekennzeichnet, dass** die zweite Stromschaltröhre (52) und die vierte Stromschaltröhre (54) beide P-Typ-Mos-Röhren sind und die erste Stromschaltröhre (51) und die dritte Stromschaltröhre (53) beide N-Typ-Mos-Röhren sind.

11. Das Energieinstrument für die Chirurgie nach Anspruch 3, **dadurch gekennzeichnet, dass** die vierte Stromschaltröhre (54) über einen Widerstand mit dem Steueranschluss der ersten Stromschaltröhre (51) verbunden ist;
wenn das Energieinstrument für die Chirurgie an eine externe Stromversorgung angeschlossen ist, ein Leiten der vierten Stromschaltröhre (54) ein Leiten der ersten Stromschaltröhre (51) auslöst, sodass die Steuereinheit (3) mit der negativen Elektrode der externen Stromversorgung verbunden wird.

12. Das Energieinstrument für die Chirurgie gemäß Anspruch 3, **dadurch gekennzeichnet, dass** die eingebaute Batterie (2) eine wiederaufladbare Batterie ist und, wenn das Energieinstrument für die Chirurgie an eine externe Stromversorgung angeschlossen ist, die wiederaufladbare Batterie über die vierte Stromschaltröhre (54) mit der externen Stromversorgung verbunden ist;
oder die eingebaute Batterie (2) eine nicht-wiederaufladbare Batterie ist.

13. Das Energieinstrument für die Chirurgie nach Anspruch 3, **dadurch gekennzeichnet, dass** die Steuereinheit (3) mit einem Gate der dritten Stromschaltröhre (53) verbunden ist, und als Reaktion auf eine Verbindung der eingebauten Batterie (2) oder einer externen Stromversorgung die Steuereinheit (3) eine Gate-Spannung der dritten Stromschaltröhre (53) detektiert und anhand der Gate-Spannung der dritten Stromschaltröhre (53) ermittelt, ob der Netzanschluss (6) mit der externen Stromversorgung verbunden ist.

14. Das Energieinstrument für die Chirurgie nach einem der Ansprüche 1 bis 13,
**dadurch gekennzeichnet, dass** das Energieinstrument für die Chirurgie ein Ultraschallskalpell, ein elektrisches Skalpell oder ein Laserskalpell ist.

## Revendications

1. Instrument à énergie pour chirurgie, qui comprend une batterie intégrée (2), une unité de commande (3), un premier circuit de consommation de puissance (41) et un second circuit de consommation de puissance (42), dans lequel le premier circuit de consommation de puissance (41) est connecté électriquement à l'unité de commande (3), et une consommation de puissance du second circuit de consommation de puissance (42) dans un état de travail est supérieure à une consommation de puissance du premier circuit de consommation de puissance (41) dans un état de travail ;
dans lequel, lorsque l'instrument à énergie pour chirurgie n'est pas connecté à une alimentation électrique externe, l'unité de commande (3) et le premier circuit de consommation de puissance (41) peuvent être déclenchés pour se connecter respectivement à une boucle avec la batterie intégrée (2), et le premier circuit de consommation de puissance (41) est configuré pour délivrer en sortie des informations d'un paramètre de condition de l'instrument à énergie pour chirurgie en réponse à une instruction de l'unité de commande (3) ;
le second circuit de consommation de puissance (42) est configuré pour rester dans un état d'arrêt de travail lorsque l'instrument à énergie pour chirurgie n'est pas connecté à une alimentation électrique externe ;
l'instrument à énergie pour chirurgie comprend en outre une touche opérationnelle (1) et un circuit de commutation, dans lequel le circuit de commutation comprend un premier tube de commutation de puissance (51) et un deuxième tube de commutation de puissance (52) avec tous deux un état initial dans une condition hors tension, dans lequel le premier tube de commutation de puissance (51) est agencé entre le premier circuit de consommation de puissance (41) et une électrode négative de la batterie intégrée (2), et le deuxième tube de commutation de puissance (52) est agencé entre le premier circuit de consommation de puissance (41) et une électrode positive de la batterie intégrée (2) ;
une borne de commande du premier tube de commutation de puissance (51) est connectée à l'électrode positive de la batterie intégrée (2) par le biais de la touche opérationnelle (1), et une borne de commande du deuxième tube de commutation de puissance (52) est connectée au premier tube de commutation de puissance (51) ; en réponse à un déclenchement de la touche opérationnelle (1), le premier tube de commutation de puissance (51) et le deuxième tube de commutation de puissance (52) sont tous deux configurés pour être convertis de la condition hors tension à une condition sous tension ;
le second circuit de consommation de puissance (42) n'est connecté ni à l'électrode positive ni à l'électrode négative de la batterie intégrée (2).

2. Instrument à énergie pour chirurgie selon la revendication 1, **caractérisé en ce que** le premier circuit de consommation de puissance (41) est l'un quelconque parmi, ou une combinaison de ceux-ci :
le premier circuit de consommation de puissance (41) est une unité d'affichage configurée pour afficher le paramètre de condition de l'instrument à énergie pour chirurgie sous la commande de l'unité de commande (3) ; et/ou
le premier circuit de consommation de puissance (41) est une unité de haut-parleur configurée pour lire des messages vocaux sous la commande de l'unité de commande (3), qui reflètent le paramètre de condition de l'instrument à énergie pour chirurgie ; et/ou
le premier circuit de consommation de puissance (41) est un dispositif de voyant lumineux configuré pour s'allumer sous la commande d'une unité de commande (3) pour refléter le paramètre de condition de l'instrument à énergie pour chirurgie.

3. Instrument à énergie pour chirurgie selon la revendication 1, **caractérisé en ce que** le circuit de commutation comprend en outre un troisième tube de commutation de puissance (53) et un quatrième tube de commutation de puissance (54) avec un état initial tous deux dans une condition hors tension, dans lequel le troisième tube de commutation de puissance (53) est agencé entre le second circuit de consommation de puissance (42) et une électrode négative de l'alimentation électrique externe, et le quatrième tube de commutation de puissance (54) est agencé entre le second circuit de consommation de puissance (42) et une électrode positive de l'alimentation électrique externe ;
une borne de commande du troisième tube de commutation de puissance (53) est connectée à l'alimentation électrique externe par le biais d'un connecteur de puissance (6), et une borne de commande du quatrième tube de commutation de puissance (54) est connectée au troisième tube de commutation de puissance (53) ; en réponse à la connexion de l'instrument à énergie pour chirurgie à l'alimentation électrique externe, le troisième tube de commutation de puissance (53) et le quatrième tube de commutation de puissance (54) sont tous deux convertis de la condition hors tension à une condition sous tension.

4. Instrument à énergie pour chirurgie selon la revendication 3, **caractérisé en ce que** le second circuit de consommation de puissance (42) est un circuit d'entraînement ou un circuit de traitement numérique.

5. Instrument à énergie pour chirurgie selon la revendication 1, **caractérisé en ce que** la touche opérationnelle (1) est un bouton dédié à la commande d'une mise en marche du premier circuit de consommation de puissance (41) ; ou,
la touche opérationnelle (1) est une touche fonctionnelle de l'instrument à énergie pour chirurgie, si l'instrument à énergie pour chirurgie est déconnecté de l'alimentation électrique externe, et la touche fonctionnelle est enfoncée, le premier tube de commutation de puissance (51) est déclenché pour conduction ; si l'instrument à énergie pour chirurgie est connecté à l'alimentation électrique externe, et la touche fonctionnelle est enfoncée, une autre fonction différente du déclenchement du premier tube de commutation de puissance (51) pour conduction est activée.

6. Instrument à énergie pour chirurgie selon la revendication 5, **caractérisé en ce qu'**une autre fonction différente du déclenchement du premier tube de commutation de puissance (51) pour conduite comprend l'une quelconque des fonctions suivantes :
la touche fonctionnelle est un bouton d'excitation, et si le bouton d'excitation est enfoncé, il activera un générateur d'énergie de l'instrument à énergie pour chirurgie afin de générer de l'énergie ; ou,
la touche fonctionnelle est un bouton de changement de puissance, et si le bouton de changement de puissance est enfoncé, il ajustera une puissance de sortie d'un générateur d'énergie de l'instrument à énergie pour chirurgie ; ou,
la touche fonctionnelle est un bouton de commande de tête de lame, et si le bouton de commande de tête de lame est enfoncé, il commandera à une extrémité d'outil de coupe de l'instrument à énergie pour chirurgie de réaliser une action de clampage.

7. Instrument à énergie pour chirurgie selon la revendication 1, **caractérisé en ce qu'**il comprend en outre une minuterie connectée électriquement à l'unité de commande (3) ;
lorsque l'instrument à énergie pour chirurgie n'est pas connecté à une alimentation électrique externe, en réponse à un déclenchement du premier circuit de consommation de puissance (41), la minuterie commence le minutage, et lorsqu'un seuil de temporisation prédéfini est atteint, le premier circuit de consommation de puissance (41) est déconnecté de la boucle avec la batterie intégrée (2).

8. Instrument à énergie pour chirurgie selon la revendication 1, **caractérisé en ce que** la touche opérationnelle (1) est configurée pour :
si la touche opérationnelle est enfoncée, le premier tube de commutation de puissance (51) reste dans la condition sous tension ;
si la touche opérationnelle (1) est relâchée, le premier tube de commutation de puissance (51) est converti de la condition sous tension à la condition hors tension.

9. Instrument à énergie pour chirurgie selon la revendication 3, **caractérisé en ce que** les bornes de commande du deuxième tube de commutation de puissance (52) et du quatrième tube de commutation de puissance (54) sont toutes deux connectées à l'électrode positive de la batterie intégrée (2) par le biais d'une résistance, et dans l'état initial, le deuxième tube de commutation de puissance (52) et le quatrième tube de commutation de puissance (54) sont tous deux dans la condition hors tension du fait que leurs tensions de grille sont toutes deux supérieures à un seuil de tension conductrice ;
en réponse à une conduction du premier tube de commutation de puissance (51), le deuxième tube de commutation de puissance (52) est mis sous tension du fait que la tension de grille de celui-ci est abaissée ; en réponse à une conduction du troisième tube de commutation de puissance (53), le quatrième tube de commutation de puissance (54) est mis sous tension du fait que la tension de grille de celui-ci est abaissée.

10. Instrument à énergie pour chirurgie selon la revendication 9, **caractérisé en ce que** le deuxième tube de commutation de puissance (52) et le quatrième tube de commutation de puissance (54) sont tous deux des tubes Mos de type P, et le premier tube de commutation de puissance (51) et le troisième tube de commutation de puissance (53) sont tous deux des tubes Mos de type N.

11. Instrument à énergie pour chirurgie selon la revendication 3, **caractérisé en ce que** le quatrième tube de commutation de puissance (54) est connecté à la borne de commande du premier tube de commutation de puissance (51) par le biais d'une résistance ;
si l'instrument à énergie pour chirurgie est connecté à une alimentation électrique externe, une conduction du quatrième tube de commutation de puissance (54) déclenche une conduction du premier tube de commutation de puissance (51), de sorte que l'unité de commande (3) est connectée à l'électrode négative de l'alimentation électrique externe.

12. Instrument à énergie pour chirurgie selon la revendication 3, **caractérisé en ce que** la batterie intégrée (2) est une batterie rechargeable, et si l'instrument à énergie pour chirurgie est connecté à une alimentation électrique externe, la batterie rechargeable est connectée à l'alimentation électrique externe par l'intermédiaire du quatrième tube de commutation de puissance (54) ;
ou la batterie intégrée (2) est une batterie non rechargeable.

13. Instrument à énergie pour chirurgie selon la revendication 3, **caractérisé en ce que** l'unité de commande (3) est connectée à une grille du troisième tube de commutation de puissance (53), et en réponse à une connexion de la batterie intégrée (2) ou d'une alimentation électrique externe, l'unité de commande (3) détecte une tension de grille du troisième tube de commutation de puissance (53) et détermine si le connecteur d'alimentation (6) est connecté à l'alimentation électrique externe par la tension de grille du troisième tube de commutation de puissance (53).

14. Instrument à énergie pour chirurgie selon l'une quelconque des revendications 1 à 13, **caractérisé en ce que** l'instrument à énergie pour chirurgie est un scalpel à ultrasons, un scalpel électrique ou un scalpel laser.
